(19) Europäisches Patentamt
European Patent Office
Office européen des brevets

(11) **EP 1 449 821 B1**

(12) **EUROPEAN PATENT SPECIFICATION**

(45) Date of publication and mention
of the grant of the patent:
**09.05.2012  Bulletin 2012/19**

(51) Int Cl.:
*C07C 51/43* *(2006.01)*   *C07C 59/13* *(2006.01)*
*C07C 59/135* *(2006.01)*   *C07B 63/00* *(2006.01)*

(21) Application number: **02785982.6**

(22) Date of filing: **29.11.2002**

(86) International application number:
**PCT/JP2002/012514**

(87) International publication number:
**WO 2003/045888 (05.06.2003 Gazette 2003/23)**

(54) **OPTICALLY ACTIVE MANDELIC ACID AND ITS DERIVATIVE, AND METHOD FOR CRYSTALLIZATION THEREOF**

"OPTISCH AKTIVE MANDELS URE UND DEREN DERIVATE, UND VERFAHREN ZU DEREN KRISTALLISIERUNG"

ACIDE MANDELIQUE OPTIQUEMENT ACTIF ET SON DERIVE, ET PROCEDE DE CRISTALLISATION CORRESPONDANT

(84) Designated Contracting States:
**AT CH DE FR GB LI NL**

(30) Priority:  **30.11.2001  JP 2001366468**

(43) Date of publication of application:
**25.08.2004  Bulletin 2004/35**

(73) Proprietor: **NIPPON SHOKUBAI CO., LTD.**
**Osaka-shi, Osaka 541-0043 (JP)**

(72) Inventor: **OKUDA, Norimasa**
**Ushiku-shi, Ibaraki 300-1207 (JP)**

(74) Representative: **Harding, Charles Thomas et al**
**D Young & Co LLP**
**120 Holborn**
**London**
**EC1N 2DY (GB)**

(56) References cited:
**EP-A- 1 148 042      EP-A- 1 160 235**
**EP-A2- 0 449 648     WO-A1-95/23139**
**US-A- 2 562 861**

**Description**

Technical Field

The present invention relates to a method of producing optically active mandelic acids, which are useful as pharmaceutical intermediates.

Background Art

Generally, mandelic acids are obtained by hydrolysis or the like of the corresponding mandelonitriles. The thus synthesized crude mandelic acids are isolated from the reaction solution through the purification steps of extraction, filtration, washing and the like. The isolated mandelic acids are then crystallized so as to obtain the crystals of mandelic acids. However, in the conventional methods, steps have been complicated, and the yield and optical purity of the obtained mandelic acid crystals have not necessarily been satisfactory.

EP-A-1148042 and US 2001/0041359 describe a process for the preparation of optically highly pure (R)- and (S)-$\alpha$-hydroxycarboxylic acids, in which either isolated or impure pure (R)- and (S)-$\alpha$-hydroxycarboxylic acids prepared by acidic hydrolysis of the (R)- and (S)-cyanohydrins are recrystallised in an aromatic hydrocarbon, optionally in the presence of a cosolvent.

EP-A-1160235, which was published after the priority date of the present application, describes a method for producing $\alpha$-hydroxycarboxylic acid which comprises hydrolysing cyanohydrin in the presence of a hydrocarbon solvent. The document further describes a method for producing optically active $\alpha$-hydroxycarboxylic acid which comprises: producing optically active cyanohydrin by reacting a carbonyl compound with HCN, using a solvent comprising at least one specified organic solvent; removing the organic solvent; and hydrolysing the remaining reaction mixture without isolating optically active cyanohydrin. The document further describes a method for producing optically active crystalline $\alpha$-hydroxycarboxylic acid comprising crystallising optically active $\alpha$-hydroxycarboxylic acid in aqueous solution, as well as optically active $\alpha$-hydroxycarboxylic acid produced by the above methods.

Disclosure of the Invention

It is the object of the present invention to provide a method of easily obtaining optically active mandelic acid with higher purity and optical purity, at a high yield, in the form of crystals that are easily handled.

The present inventors have found that the above problems can be solved by crystallizing optically active mandelic acids from an aqueous solution in the presence of an alkali and an organic solvent non-miscible with water, in which the above optically active mandelic acids are hardly soluble, thereby completing the present invention.

That is to say, the present invention includes the following features:

(1) A method of crystallizing optically active mandelic acids characterized in that it comprises adding alkali to an aqueous solution comprising optically active mandelic acids and mineral wherein the amount of said alkali is 0.2 to 0.9 equivalence with said mineral acid in the aqueous solution, and then crystallizing the optically active mandelic acids from the above aqueous solution.

(2) A method of crystallizing optically active mandelic acids characterized in that it comprises adding alkali to an aqueous solution comprising optically active mandelic acids and mineral acid, wherein the amount of said alkali is 0.2 to 0.9 equivalence with said mineral acid in the aqueous solution, mixing into the above aqueous solution an organic solvent non-miscible with water, in which the above mandelic acids are hardly soluble, and then crystallizing the optically active mandelic acids from the above aqueous solution.

(3) The method according to (2) above, wherein the solubility of the above organic solvent in water at 20°C is less than 1% by weight, and the solubility of the above mandelic acids in the organic solvent at 20°C is less than 2% by weight.

The present invention relates to a method of crystallizing optically active mandelic acids characterized in that it comprises adding alkali to an aqueous solution comprising optically active mandelic acids and mineral acid wherein the amount of said alkali is 0.2 to 0.9 equivalence with said mineral acid in the aqueous solution, and then crystallizing the optically active mandelic acids from the above aqueous solution.

Examples of optically active mandelic acids used in the present invention include mandelic acid and derivatives thereof such as that having a substituent on the benzene ring of mandelic acid. Specific examples of the above substituent include straight chain or branched chain alkyl groups having 1 to 5 carbon atoms such as a methyl or ethyl group; halogens such as chlorine, bromine or fluorine; and cyano groups. Specific examples of the derivative of mandelic acid having the above substituent include 2-chloromandelic acid, 3-chloromandelic acid, and 4-chloromandelic acid. Moreover, either an R form or S form of optically active mandelic acid may be used in the present invention.

In the present invention, the crystallization of mandelic acids is carried out as follows:

Mandelic acids are dissolved in water, so as to prepare an aqueous solution comprising them. This time, the operation may appropriately be carried out while water is heated, so that the mandelic acids are completely dissolved therein. For example, when mandelic acids are obtained by hydrolysis of the corresponding mandelonitriles, the generated crude mandelic acids may not be isolated from the reaction solution after completion of the reaction, but an appropriate amount of water may be added to the solution, and the thus obtained solution may be used as a solution comprising mandelic acids. For example, the acid catalyzed hydrolysis of mandelonitriles is carried out using mineral acid (e.g., hydrochloric acid, sulfuric acid, nitric acid, boric acid, phosphoric acid, perchloric acid, etc.), and an aqueous solution obtained by the hydrolysis can be directly used. Since the solution obtained by the acid catalyzed hydrolysis contains optically active mandelic acids and mineral acid generated as a result of the hydrolysis reaction, it is acidic. In the present invention, alkali is added to the thus obtained aqueous solution containing optically active mandelic acids and mineral acid to carry out partial neutralization (that is, incomplete neutralization), and thereafter, the mandelic acids are crystallized. The term "partial neutralization" is used in the present specification to mean that neutralization is carried out, using 0.2 to 0.9 equivalence of alkali with said mineral acid existing in an aqueous solution. Examples of alkali include potassium hydroxide and sodium hydroxide. The additive amount of alkali is a 0.2 to 0.9 equivalence, and preferably a 0.4 to 0.9 equivalence, with the mineral acid in the aqueous solution.

The present inventors have found that the recovery rate and purity of optically active mandelic acids crystals can be improved by carrying out the above-described partial neutralization. The reason is not known conclusively, but it seems to be due to (1) decomposition of ester compounds, i.e., dimers such as mandelic acids, and (2) the effect of salt concentration.

The present inventors have found that the above ester compounds such as dimers are easily decomposed and dissociated in a basic aqueous solution, so that they become monomers of mandelic acids. However, if an entire aqueous solution from which mandelic acids are crystallized is basic, the mandelic acids form salts. As a result, solubility becomes too high and it becomes difficult to crystallize mandelic acids therefrom, thereby decreasing the recovery rate of the crystals. Thus, the present inventors have made further intensive studies, and as a result, they have found that if 0.2 to 0.9 equivalence of alkali is added to an aqueous solution to such an extent that it does not completely neutralize an entire aqueous solution, and crystallization is then carried out, a majority of the above dimers can be dissociated and decomposed without increasing the solubility of mandelic acids in water, thereby completing the present invention. The term "partial neutralization" is used in the present specification to mean that as described above, 0.2 to 0.9 equivalence of alkali is added to an acidic aqueous solution, such that it does not completely neutralize the solution.

By carrying out such partial neutralization, mineral acid is reacted with alkali in an aqueous solution, so as to form inorganic salts. For example, the addition of sodium hydroxide into an aqueous solution containing hydrochloric acid as mineral acid forms NaCl. Generation of inorganic salts by addition of alkali increases salt concentration in an aqueous solution, and thus, solubility of mandelic acids decreases. It was found that this enables the improvement of the recovery rate of mandelic acids during crystallization.

It is considered that the recovery rate of mandelic acids crystals can be improved by the synergism of the above two effects, that is, (1) decomposition of ester compounds, i.e., dimers such as mandelic acids, and (2) the effect of salt concentration.

An organic solvent "non-miscible with water" means in the present specification an organic solvent separable from a water phase for phase separation. The solubility of such an organic solvent in water at 20°C is preferably less than 1% by weight. Moreover, an organic solvent "in which mandelic acids are hardly soluble" means herein, for example, an organic solvent, wherein the solubility of the mandelic acids is less than 2% by weight at 20°C, and preferably less than 0.5% by weight at 20°C.

By carrying out crystallization with addition of such an organic solvent, by-products generated as a result of the hydrolysis of mandelonitriles are deposited to an organic solvent phase. Accordingly, when compared with the case of crystallizing only from an aqueous solution, optically active mandelic acids crystals with a higher purity and less color can be obtained. Moreover, when mandelic acids are crystallized only from an aqueous solution, generally, only micro-particle crystals can be obtained, but when crystallization is carried out in the presence of an organic solvent, granular crystals with the ease of handling can be obtained.

A hydrocarbon solvent is an example of the organic solvent non-miscible with water in which the above mandelic acids are hardly soluble. Examples of such a hydrocarbon solvent include straight- or branched-chain hydrocarbons, cyclic hydrocarbons with or without side chains, and chain hydrocarbons with the substitution of the above cyclic hydrocarbon group. Moreover, these hydrocarbons may have unsaturated bonds in their molecules. Some typical hydrocarbon solvents will be described below.

Examples of a straight- or branched-chain hydrocarbon solvent include pentane, hexane, heptane, octane, and their structural isomers including chain hydrocarbons containing 5 to 16 carbon atoms such as 2-methylpentane or 3-methylpentane. Examples of a cyclic hydrocarbon with or without a side chain include saturated monocyclic hydrocarbons containing 6 to 16 carbon atoms such as cyclopentane, cyclohexane, and their structural (constitutional) isomers, for example, methylcyclopentane or methylcyclohexane; and aromatic hydrocarbons such as benzene, toluene, trimethylbenzene, o-xylene, m-xylene, p-xylene, or an isomeric mixture of xylene.

Of these hydrocarbon solvents, aromatic hydrocarbon solvents such as benzene, toluene or p-xylene are preferable, and toluene is more preferable. In addition, a mixed solvent obtained by combination of two or more types of solvents may also be used in the present invention.

When the above organic solvent is added to an aqueous solution, the ratio between the solution and the organic solvent is preferably 1:0.05 to 1:1 in terms of weight ratio. When the mixture of the mandelic acid aqueous solution and the organic solvent is cooled, the crystals of mandelic acids are deposited. The cooling temperature is not particularly limited, as long as it is below the temperature at which the above mandelic acid aqueous solution becomes saturated. It is preferably 30°C or lower, and more preferably 20°C or lower. Too-rapid cooling is not appropriate, but it is preferable to cool the mixture at a cooling rate of 0.5°C/min or lower. Subsequently, the deposited optically active mandelic acids crystals are filtrated, and the filtrated crystals are washed with water and an organic solvent such as toluene. The optically active mandelic acids crystals obtained by filtration generally contain 5.0% or more water by weight. Accordingly, the crystals are dried until their water content becomes less than 5% by weight, and preferably between 0.01 % and 0.2% by weight.

When the above-described partial neutralization is carried out in combination with the addition of an organic solvent, the partial neutralization is preferably carried out as follows: When an organic solvent is added to a mandelic acid aqueous solution followed by leaving at rest, the solution may be separated into syrupy high-concentration and low-concentration phases at times. In this case, most of the dimers of mandelic acids are distributed in the high-concentration phase. So, this high-concentration phase is once separated. Alkali is then added to the separated phase, so that the dimers of mandelic acids are dissociated. The solution of this high-concentration phase is basic, but when the high-concentration phase is mixed with the low-concentration phase again, the entire solution becomes acidic, and thus, partial neutralization is carried out. Subsequently, crystallization is carried out so as to obtain the crystals of mandelic acids.

Thus, mandelic acids are crystallized so as to obtain the crystals of mandelic acids. The crystals show a particle size distribution, in which their particle size is frequently distributed in a range of 300 $\mu$m to 1,000 $\mu$m.

According to the crystallization method of the present invention, mandelic acids crystals can easily be obtained at a good recovery rate as granular crystals. When compared with crystals obtained by the conventional method, the mandelic acid crystals obtained by the present method have an extremely high optical purity and a high filling (packing) density, and the ease of handling, having a relatively uniform particle size. For example, when optically active 2-chloromandelic acids are used as optically active mandelic acids, the obtained crystals of optically active 2-chloromandelic acids have a filling density of 0.55 g/cm$^3$ or higher, and the crystals do not cause many problems regarding ease of dispersibility, or ease of adhesiveness to the wall of a container. The reason why the crystals obtained by the present method do not cause the above problems is not clear. According to the conventional method involving deposition of crystals from an organic solvent, however, since impurities existing in the organic solvent solution affect the solubility and crystallization of optically active mandelic acids, only crystals having a low filling density are obtained. Moreover, since the difference of the solubility caused by temperature is often smaller than in the case of the use of water, it is difficult to obtain high-purity crystals at a high recovery rate.

The optically active mandelic acids crystals produced by the process of the present invention have a filling density of 0.55 g/cm$^3$ or higher, and preferably 0.60 g/cm$^3$ or higher. 60% or more by weight, and preferably 65% or more by weight, of these crystals are distributed in a particle size range of 300 $\mu$m to 1,000 $\mu$m. These crystals are significantly improved in terms of the ease of handling in operations, such as filling a container therewith, transfer to another container, or weighing. Moreover, the crystals are characterized in that they hardly disperse or adhere to the wall of a container. Furthermore, since the crystals have a high filling density, they take up less space during their transportation or storage, so that transportation or equipment costs can be reduced. Thus, the optically active mandelic acids crystals of the present invention are highly useful industrially.

The optically active mandelic acids crystals produced by the process of the present invention are obtained, for example, by preparation from a solution containing the water of optically active mandelic acids, variously selecting crystallization means and conditions. In particular, the application of the above-described crystallization method of the present invention is preferable to efficiently obtain granular crystals with a high optical purity and a relatively uniform particle size.

The Japanese Patent Application No. 2001-366468 is a priority document of the present application.

Best Mode for Carrying Out the Invention

The present invention is further described in the following examples and comparative examples. These examples are provided for illustrative purposes only, and are not intended to limit the scope of the invention.

(Example 1) (reference)

51.3 g of (R)-2-chloromandelonitrile with an optical purity of 96%ee ((R)-2-chloromandelonitrile: 0.30 mol) and 96.0 g of 35% hydrochloric acid (HCl: 0.92 mol) were placed in a 200 ml flask, and a hydrolysis reaction was carried out, stirring at 60°C for 7 hours. After completion of the reaction, 55.0 g of water and 15.0 g of toluene as an organic solvent were added thereto followed by stirring at 55°C for 10 minutes. The thus obtained solution consisting of water and toluene phases was cooled to 10°C at a constant cooling rate over 4 hours while stirring, and the solution was kept at 10°C for 2 hours. The deposited crystals were filtrated, and the filtrated crystals were washed with 30 g of water and then with 30 g of toluene. The obtained crystals were dried under reduced pressure to obtain 51.2 g of (R)-2-chloromandelic acid crystals. The purity of (R)-2-chloromandelic acid was 99.5% and the optical purity was 99.9%ee by HPLC. The yield of the (R)-2-chloromandelic acid recovered was 91.0%.

(Example 2) (reference)

51.5 g of (R)-2-chloromandelic acid crystals was obtained in the same manner as in Example 1 with the exception that benzene was used instead of toluene as an organic solvent to add after completion of the hydrolysis reaction. The purity of (R)-2-chloromandelic acid was 99.3% and the optical purity was 99.8%ee by HPLC. The yield of the (R)-2-chloromandelic acid recovered was 91.3%.

(Example 3) (reference)

51.5 g of (R)-2-chloromandelic acid crystals was obtained in the same manner as in Example 1 with the exception that p-xylene was used instead of toluene as an organic solvent to add after completion of the hydrolysis reaction. The purity of (R)-2-chloromandelic acid was 99.3% and the optical purity was 99.8%ee by HPLC. The yield of the (R)-2-chloromandelic acid recovered was 91.3%

(Comparative example 1)

52.7 g of (R)-2-chloromandelic acid crystals was obtained in the same manner as in Example 1 with the exception that toluene was not added after completion of the hydrolysis reaction. The purity of (R)-2-chloromandelic acid was 98.3% and the optical purity was 99.0%ee by HPLC. The yield of the (R)-2-chloromandelic acid recovered was 92.0%.

(Comparative example 2)

A hydrolysis reaction was carried out as described in Example 1. After completion of the reaction, 100 g of ethyl acetate was added to the reaction product, and the mixture was shaken in a separatory funnel. Thereafter, the organic phase was separated from the water phase. The water phase was extracted with 100 g of ethyl acetate, and the obtained organic phase was mixed with the organic phase obtained by the above operation followed by washing with 30 g of water. The thus obtained organic phase was exsiccated under reduced pressure, using an evaporator. The obtained crude crystals were dissolved in 400 g of toluene-ethyl acetate (with a weight ratio of 9:1) at 70°C, and then cooled to 10°C at a constant cooling rate over 4 hours, while stirring. Thereafter, the solution was kept at 10°C for 2 hours. The deposited crystals were filtrated, and the filtrated crystals were then washed with 30 g of toluene. The obtained crystals were dried under reduced pressure to obtain 48.2 g of (R)-2-chloromandelic acid crystals. The purity of (R)-2-chloromandelic acid was 99.3% and the optical purity was 99.9%ee by HPLC. The yield of the (R)-2-chloromandelic acid recovered was 85.5%.

(Example 4)

51 g of (R)-2-chloromandelonitrile with an optical purity of 96.0%ee ((R) form: 0.30 mol) and 96 g of 35% hydrochloric acid (HCl: 0.92 mol) were placed in a 200 ml flask, and a hydrolysis reaction was carried out, stirring at 60°C for 7 hours. After completion of the reaction, 15 g of toluene was added thereto followed by stirring at 55°C for 10 minutes. Then, the obtained solution was left at rest for 10 minutes while its temperature was kept at 55°C. Thereafter, a high-concentration (R)-2-chloromandelic acid phase that was separated and accumulated at the bottom was collected, and 67 g of a 18 wt % sodium hydroxide solution was added thereto. Thereafter, the phase was mixed with the remaining phases (a toluene phase and a low-concentration water phase), and the temperature of the mixed phase was set at 55°C. This water/toluene phase's mixed solution was cooled to 10°C at a constant cooling rate over 4 hours while stirring, and the solution was kept at 10°C for 2 hours. The deposited mandelic acid crystals were filtrated, and the filtrated crystals were washed with 30 g of water and then with 30 g of toluene. The water content of the obtained (R)-2-chloromandelic acid crystal wet product was 10% by weight. Thereafter, the (R)-2-chloromandelic acid crystal wet product was dried under reduced pressure to obtain 52.5 g of dry (R)-2-chloromandelic acid crystals (yield: 93.2%). The purity of the obtained dry (R)-2-chloromandelic acid crystals was 99.6% and the optical purity was 99.9%ee by HPLC. The water content of the crystal was measured by the Karl Fischer method.

(Example 5)

52.9 g (yield: 94.0%) of dry (R)-2-chloromandelic acid crystals was obtained in the same manner as in Example 4 with the exception that 74.3 g of a sodium hydroxide solution with a concentration of 26 wt % was used. The purity of the obtained dry (R)-2-chloromandelic acid crystal was 99.6% and the optical purity was 99.9%ee by HPLC.

(Example 6)

53.1 g (yield: 94.3%) of dry (R)-2-chloromandelic acid crystals was obtained in the same manner as in Example 4 with the exception that 15 g of p-xylene was added instead of 15 g of toluene. The purity of the obtained dry (R)-2-chloromandelic acid crystal was 99.5% and the optical purity was 99.8%ee by HPLC.

The results of Examples 1 to 6 and Comparative examples 1 and 2 are shown in Table 1.

Table 1

| No. | Solvents for crystallization | Partial neutralization | Yield of R-2CMA[1] (%) | Purity of carboxylic acid[2] (%) | Optical purity (%e.e.) | Content of dimers (%) |
|---|---|---|---|---|---|---|
| Example 1 (ref) | Water/ toluene | No | 91.0 | 99.5 | 99.9 | 0.40 |
| Example 2 (ref) | Water/ benzene | No | 91.3 | 99.3 | 99.8 | 0.58 |
| Example 3 (ref) | Water/ p-xylene | No | 91.3 | 99.3 | 99.8 | 0.61 |
| Example 4 | Water/ toluene | NaOH | 93.2 | 99.6 | 99.9 | 0.29 |
| Example 5 | Water/ toluene | NaOH | 94.0 | 99.6 | 99.9 | 0.25 |
| Example 6 | Water/ p-xylene | NaOH | 94.3 | 99.5 | 99.8 | 0.36 |
| Comparative example 1 | Water only | No | 92.0 | 98.3 | 99.0 | 0.92 |
| Comparative example 2 | Toluene/ethyl acetate | No | 85.5 | 99.3 | 99.9 | 0.18 |

1) R-2CMA: (R)-2-chloromandelic acid. Yield is calculated in consideration of purity and optical purity.
2) Purity of carboxylic acid represents the purity of the obtained 2-chloromandelic acid (R form and S form) (HPLC).

Subsequently, the (R)-2-chloromandelic acid crystals obtained in Examples 1 to 6 and Comparative examples 1 and 2 were measured regarding appearance, water content, filling density, compression rate, and particle size distribution. Each of the above items was measured according to the following methods.

Measurement of water content

Water content was measured by the Karl Fischer method.

Measurement of filling (packing) density

10 to 20 g of each of the crystals obtained in the above Examples and Comparative examples was weighed, and it was passed through a funnel with a diameter of 20 mm to naturally drop into a 50 ml measuring cylinder, so that the weight and volume of the crystals were measured and the filling density was calculated.

Measurement of compression rate

An approximately 7-cm$^3$ chloromandelic acid crystal was passed through a funnel with a diameter of 9 mm, and it was naturally dropped into a 10 ml measuring cylinder from a height of 20 cm, so that the volume of the crystals was measured (volume 1). Thereafter, the crystals in the measuring cylinder were compressed with a glass rod until their volume was not reduced, and the volume after the compression was measured (volume 2). Compression rate was calculated according to the following formula.

$$\text{Compression rate} = (\text{volume 1} - \text{volume 2}) / \text{volume 1}$$

Measurement of particle size distribution

The crystals were shifted using a microtype electromagnetic vibrating shifter (M-2 type, manufactured by Tsutsui Rikagaku Kikai Co., Ltd.), and their weight was measured, so that their particle size distribution was obtained. 20 g of the crystals was placed on a sieve with a mesh of 1,000 $\mu$m, and another sieve with a mesh of 300 $\mu$m was placed below the above sieve followed by vibration for 10 minutes.

The properties of the crystals obtained in Examples 1 to 6 and Comparative examples 1 and 2 are shown in Table 2.

Table 2

| No. | Appearance | Water content (wt %) | Filling density (g/cm$^3$) | Compression rate | Particle size distribution (weight %) | | |
|---|---|---|---|---|---|---|---|
| | | | | | <300 $\mu$m | 300 to 1,000 $\mu$m | 1,000 $\mu$m< |
| Example 1 (ref) | Granular | 0.046 | 0.65 | 0.03 | 15.5 | 73.5 | 11.0 |
| Example 2 (ref) | Granular | 0.049 | 0.63 | 0.03 | 18.2 | 73.2 | 8.0 |
| Example 3 (ref) | Granular | 0.058 | 0.63 | 0.03 | 16.6 | 71.0 | 12.4 |
| Example 4 | Granular | 0.062 | 0.68 | 0.03 | 17.3 | 72.2 | 10.5 |
| Example 5 | Granular | 0.076 | 0.68 | 0.03 | 18.4 | 73.1 | 8.5 |
| Example 6 | Granular | 0.080 | 0.64 | 0.03 | 19.0 | 71.3 | 9.7 |
| Comparative example 1 | Fine powder | 0.110 | 0.68 | 0.04 | 46.2 | 46.5 | 7.3 |
| Comparative example 2 | Scale | 0.005 | 0.29 | 0.62 | 24.6 | 71.3 | 4.1 |

(Example 7)

The ease of handling of the chloromandelic acid crystals obtained in Examples 1 to 6 and Comparative examples 1 and 2 was evaluated. The ease of handling was evaluated by measuring (1) adhesiveness and (2) fluidity. Each of these items were measured as follows:

(1) Adhesiveness

Chloromandelic acid crystals were naturally dropped in a dry test tube having an inside diameter of 16 mm, so that the height of the crystals became approximately 10 cm. Thereafter, the test tube containing chloromandelic acid crystals was slowly inverted, so that the test tube was completely turned upside down and the crystals were dropped. Thereafter, the weight of the test tube was measured, and the weight of chloromandelic acid crystals adhered to the inner wall of the test tube was obtained. The ratio (%) of the weight of the adhering crystals to the weight of the initially filled chloromandelic acid crystals was defined as an index of adhesiveness.

(2) Flowability

Glass funnels obtained by connecting glass tubes (cylindrical portions) having inside diameters of 4, 5, 6, 8, 9 and 11 mm with the edges of cones with apical angles of 60° were used. The outlet of each funnel (outlet of cylindrical portion) was closed. Each funnel was filled with 15 g of chloromandelic acid crystals by naturally dropping the crystals little by little, in such a manner that they did not block in the cylindrical portion. During this process, the funnel should not be shaken, and chloromandelic acid crystals should not be pushed into the funnel by compression. Thereafter, each funnel containing chloromandelic acid crystals was left at rest, the outlet of the cylindrical portion of each funnel was opened, and the crystals were naturally dropped from the outlets. This operation was carried out 5 times, using each of the above described funnels having cylindrical portions with inside diameters of 4, 5, 6, 8, 9 and 11 mm. Flow limit diameter (mm) was defined as a minimum inside diameter capable of naturally dropping the total amount of chloromandelic acid crystals in all of the above 5 tests with no blockage in the funnels. The flow limit diameter was defined as an index of fluidity.

The adhesion rate and flow limit diameter of the chloromandelic acid crystals obtained in Examples 1 to 6 and Comparative examples 1 and 2 were measured according to the above-described methods. The results are shown in Table 3.

Table 3

| No. | Solvents for crystallization | Partial neutralization | Adhesion rate (%) | Flow limit diameter (mm) |
|---|---|---|---|---|
| Example 1 (ref) | Water/toluene | No | 0.057 | 5 |
| Example 2 (ref) | Water/ benzene | No | 0.061 | 5 |
| Example 3 (ref) | Water/p-xylene | No | 0.058 | 5 |
| Example 4 | Water/toluene | NaOH | 0.110 | 5 |
| Example 5 | Water/toluene | NaOH | 0.130 | 5 |
| Example 6 | Water/p-xylene | NaOH | 0.140 | 5 |
| Comparative example 1 | Water only | No | 0.830 | 9 |
| Comparative example 2 | Toluene/ethyl acetate | No | 0.500 | 11 |

Industrial Applicability

The present invention provides a method of easily obtaining, at a high yield, optically active mandelic acids crystals having a high filling density, a high purity and optical purity, and a particle size that is easily handled. Moreover, since optically active mandelic acid crystals obtained by the method of the present invention have an appropriate particle size distribution and hardly adhere to a container or the like, loss due to dispersion or transfer to another container can significantly be reduced. They are also excellent in terms of fluidity and ease of handling. Furthermore, in the present invention, since crystallization is carried out by adding alkali to an aqueous solution containing optically active mandelic acids and mineral acid for partial neutralization, the yield and purity of optically active mandelic acids can be improved. Still further, since acid in the aqueous solution is partially neutralized, corrosion of equipment can be reduced.

**Claims**

1. A method of crystallizing optically active mandelic acids **characterized in that** it comprises adding alkali to an aqueous solution comprising optically active mandelic acids and mineral acid, wherein the amount of said alkali is 0.2 to 0.9 equivalence with said mineral acid in the aqueous solution, and then crystallizing the optically active mandelic acids from said aqueous solution.

2. A method of crystallizing optically active mandelic acids **characterized in that** it comprises adding alkali to an aqueous solution comprising optically active mandelic acids and mineral acid, wherein the amount of said alkali is 0.2 to 0.9 equivalence with said mineral acid in the aqueous solution, mixing into said aqueous solution an organic solvent non-miscible with water, in which said mandelic acids are hardly soluble, and then crystallizing the optically active mandelic acids from said aqueous solution.

3. The method according to claim 2, wherein the solubility of said organic solvent in water at 20°C is less than 1% by weight, and the solubility of said mandelic acids in the organic solvent at 20°C is less than 2% by weight.

**Patentansprüche**

1. Verfahren zum Kristallisieren von optisch aktiven Mandelsäuren, **dadurch gekennzeichnet, dass** es umfasst, dass man zu einer optisch aktive Mandelsäuren und Mineralsäure umfassenden wässrigen Lösung Alkali gibt, wobei die Menge an Alkali 0,2 bis 0,9 Äquivalente zu der Mineralsäure in der wässrigen Lösung beträgt, und dann die optisch aktiven Mandelsäuren aus der wässrigen Lösung kristallisiert.

2. Verfahren zum Kristallisieren von optisch aktiven Mandelsäuren, **dadurch gekennzeichnet, dass** es umfasst, dass man zu einer optisch aktive Mandelsäuren und Mineralsäure umfassenden wässrigen Lösung Alkali zugibt, wobei die Menge an Alkali 0,2 bis 0,9 Äquivalente zu der Mineralsäure in der wässrigen Lösung beträgt, in die wässrige Lösung ein mit Wasser nicht mischbares organisches Lösungsmittel, in welchem die Mandelsäuren kaum löslich sind, einmischt und dann die optisch aktiven Mandelsäuren aus der wässrigen Lösung kristallisiert.

3. Verfahren nach Anspruch 2, wobei die Löslichkeit des organischen Lösungsmittels in Wasser bei 20 °C weniger als 1 Gew.-% beträgt und die Löslichkeit der Mandelsäuren in dem organischen Lösungsmittel bei 20 °C weniger als 2 Gew.-% beträgt.

**Revendications**

1. Procédé de cristallisation d'acides mandéliques optiquement actifs **caractérisé en ce qu'**il comprend l'addition d'alcali à une solution aqueuse comprenant des acides mandéliques optiquement actifs et un acide minéral, où la quantité dudit alcali est 0,2 à 0,9 équivalence avec ledit acide minéral dans la solution aqueuse, puis la cristallisation des acides mandéliques optiquement actifs dans ladite solution aqueuse.

2. Procédé de cristallisation d'acides mandéliques optiquement actifs **caractérisé en ce qu'**il comprend l'addition d'alcali à une solution aqueuse comprenant des acides mandéliques optiquement actifs et un acide minéral, où la quantité dudit alcali est 0,2 à 0,9 équivalence avec ledit acide minéral dans la solution aqueuse, le mélange dans ladite solution aqueuse d'un solvant organique non miscible à l'eau, où lesdits acides mandéliques sont peu solubles, puis la cristallisation des acides mandéliques optiquement actifs dans ladite solution aqueuse.

3. Procédé selon la revendication 2 où la solubilité dudit solvant organique dans l'eau à 20°C est inférieure à 1 % en poids, et la solubilité desdits acides mandéliques dans le solvant organique à 20°C est inférieure à 2 % en poids.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1148042 A
- US 20010041359 A
- EP 1160235 A
- JP 2001366468 A